# EUROPEAN PATENT APPLICATION

(11) **EP 0 911 042 A1**
(43) Date of publication of application: **28.04.1999**
(21) Application number: 98118675.2
(22) Date of filing: 02.10.1998
(51) Int. Cl.: A61M 1/16, A61M 1/34

(54) **Equipment for dialysis treatment**

(30) Priority: 07.10.1997 IT BO970602
(71) Applicant: BELLCO S.p.A., I-41037 Mirandola (IT)
(72) Inventor: Biserni Mario, Mirandola 41037 (IT)
(74) Representative: Cerbaro, Elena

(57) **Abstract**

Equipment for dialysis treatment includes a dialyser (3) in which there are two channels delimited by a membrane (4), the first for the extracorporeal blood and the second for a dialysis liquid, a first duct (5) along which the dialysis liquid flows towards the second channel of the dialyser (3), first pump means (6) for controlling the flow of the dialysis liquid along the first duct (5) a second duct (7) along which the liquid output from the second channel of the dialyser (3) flows towards a discharge output, and second pump means (6) for controlling the flow of the dialysis liquid along the second duct (7). The main characteristic of the apparatus is that the first and second pump means comprise at least one volumetric pump (6).

## Description

The present invention concerns equipment for dialysis treatment.

As is known, one of the main problems of the existing equipment for dialysis treatment is the difficulty of detecting the weight loss of the patient during the treatment. In particular, this equipment has a series of peristaltic pumps the activation of which causes liquid to flow along the various ducts of the apparatus, and sophisticated and expensive devices which detect the weight loss of the patient by measuring the flow rates of the fluids along the aforesaid ducts. These flow rates are measured using invasive equipment, that is, equipment that comes into contact with the fluids circulating in the ducts of the apparatus. Because of this, after each dialysis treatment, the aforesaid equipment must be subjected to a series of sterilisation procedures so that they can be reutilised in a subsequent treatment. The equipment for measuring the flow rates could be unnecessary if the peristaltic pumps were extremely precise, so that in order to detect the weight loss of the patient it would be sufficient to detect the functional characteristics of the pumps and from these calculate the flow rates caused thereby. However, these peristaltic pumps are unfortunately insufficiently precise for a dialysis treatment in which the weight loss of the patient must be followed with particular care during the entire dialysis treatment in order to avoid cardiac imbalance arising in the patient.

The object of the present invention is to provide equipment for dialysis treatment that is free from the disadvantages indicated above.

According to the present invention there is provided equipment for dialysis treatment of the type including:
a dialyser in which a membrane delimits two channels, the first for the extracorporeal blood and the second for a dialysis liquid;
a first duct along which the said dialysis liquid flows towards the second channel of the said dialyser;
first pump means for controlling the flow of the said dialysis liquid along the said first duct;
a second duct along which the liquid output from the said second channel of the said dialyser flows towards a discharge outlet;
second pump means for controlling the flow of the said dialysis liquid along the said second duct;
   characterised in that at least one of the said first and second pump means comprises at least one volumetric pump.

For a better understanding of the present invention a preferred embodiment will now be described, purely by way of non-limitative example, with reference to the accompanying drawings in which:
Figure 1 is a block diagram of dialysis equipment produced according to the present invention;
Figure 2 is a view of one element of the equipment of Figure 1; and
Figures 3 to 6 are diagrams illustrating the operation of the element of Figure 2.

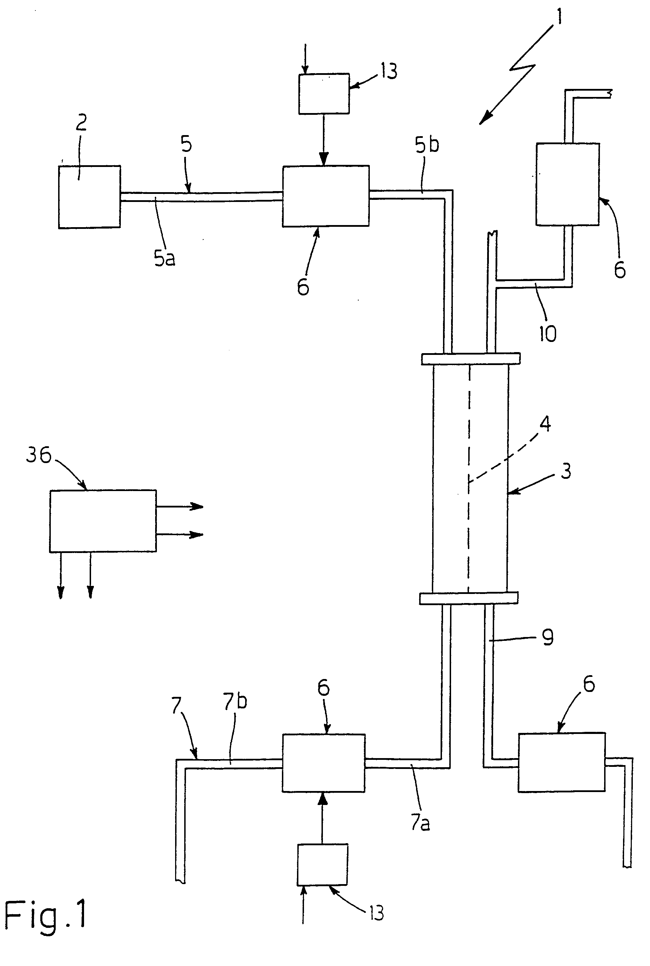

According to Figure 1, the reference numeral 1 generally indicates equipment for dialysis treatments. This apparatus 1 essentially comprises three parts, a first part concerns the preparation of the dialysis bath, the second part concerns the utilisation of the dialysis bath, and the third part concerns the discharge of the fluid output from the second part and the monitoring of the weight loss of the patient.

The second part of the equipment 1 includes a dialyser 3 of known type in which there are two channels delimited by a membrane 4, the first for the extracorporeal blood and the second for the dialysis liquid; the extracorporeal blood flowing along a duct 9.

The first part of the apparatus 1 includes, as illustrated schematically with a block 2, a source of dialysis liquid formed by mixing water and one or more concentrated salines. The equipment 1 further includes a duct 5 having a first end connected to the block 2 and a second end connected to the aforesaid second channel of the dialyser 3, through which duct the dialysis liquid is released. A pump 6 is installed along the duct 5 to channel the dialysis liquid towards the dialyser 3.

The third part of the equipment 1 includes a duct 7 connected to a first end of the aforesaid second channel of the dialyser 3 and a second pump 6 installed along the duct 7; the second end of the duct 7 is connected to a discharge outlet, not illustrated, and the second pump 6 channels the dialysis liquid discharging from the dialyser 3 towards the discharge outlet.

The equipment 1 described above with reference to Figure 1 is of known type and, in particular, is apparatus for conventional dialysis treatments from which originates apparatus related to haemofiltration, haemodiafiltration or haemodiaultrafiltration treatments. Although applied to conventional equipment, the inventive concepts of the present invention, which will be described below, are to be understood as able to be extended to any kind of dialysis equipment such as that described above in which it is in any case necessary to channel fluids, being the dialysis liquid and/or the blood and/or the infusion liquid.

The main inventive characteristic of the present invention is the utilization of a volumetric pump to channel the fluid in question and, in this embodiment, to channel the dialysis liquid. In addition, the volumetric pump has two main characteristics which enable its correct and effective application in dialysis equipment. The first characteristic is that motorisation means are releasably coupled with the volumetric pump, and the second characteristic is that the volumetric pump is of the "disposable" type so that after each treatment the pump is not used again and is therefore disposed of.

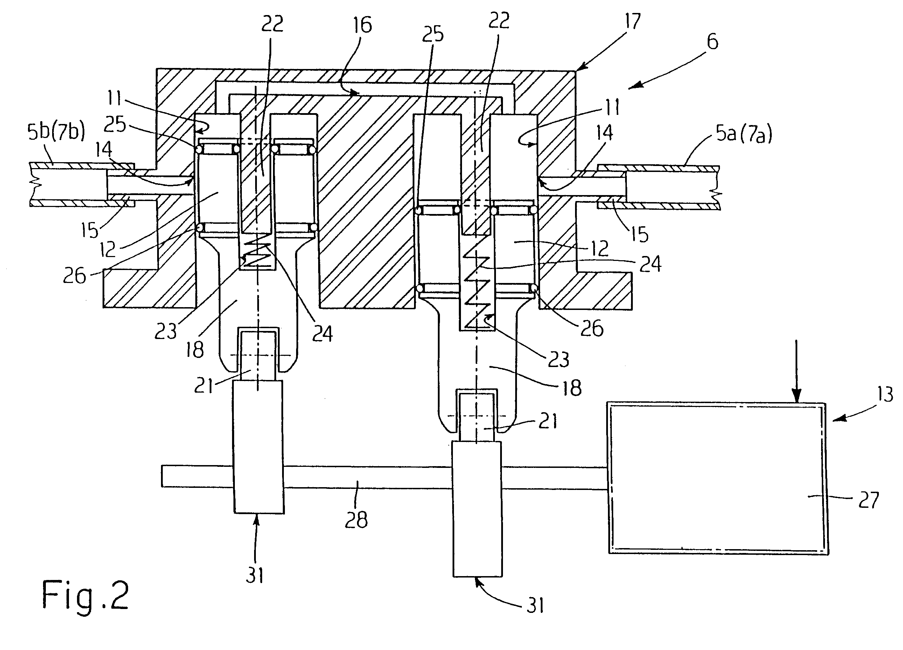

Both of the pumps, indicated 6, are volumetric pumps, and one pump 6 is illustrated in section in Figure 2. The pump 6 includes two cylinders 11 having longitudinal, parallel axes, and two pistons 12 which, by means of motorisation means 13, slide inside the cylinders 11 between an upper dead point and a lower dead point and vice versa. Each cylinder 11 has a mouth 14 and a connection 15 in which the end of a portion of one of the ducts 5 or 7 is fitted. In particular, the duct 5 has a first portion 5a between block 2 and the first pump 6, and a second portion 5b between the first pump 6 and the dialyser 3, and the duct 7 has a first portion 7a between the dialyser 3 and the second pump 6, and a second portion 7b between the second pump 6 and the discharge outlet. A channel 16 is defined in each pump 6, between the head portions of the cylinders 11.

With reference to Figure 2, the cylinders 11 form part of a head body 17 preferably formed in one piece of plastics material. Two blind cylindrical holes are formed in the head body 17, which constitute the cylinders 11 and the channel 16. The pistons 12 are also formed from plastics material and have a rod 18 which extends out of the cylinder 11 and supports a roller 21 freely mounted at its free end. A guide is formed between each cylinder 11 and the related piston 12, comprising a pin 22 which originates from the base wall of the cylinder 11 and which engages a blind hole 23 formed in the head of the piston 12; preloaded spring means 24 are installed between the base wall of the hole 23 and the free end of the pin 22, the action of which consists of pushing the piston 12 towards the outside of the cylinder 11. Finally, each piston 12 has a seal 25 mounted at the head of the piston 12, and a seal 26 mounted at the lower portion of the piston 12. It should be emphasised that the mouths 14 of the two cylinders 11 are at the same level and that when the pistons 12 are at their upper dead point, the seal 26 is at a lower level than the mouth 14. When the pistons 12 are at their lower dead point, the mouth 14 is in communication with the corresponding cylinder 11.

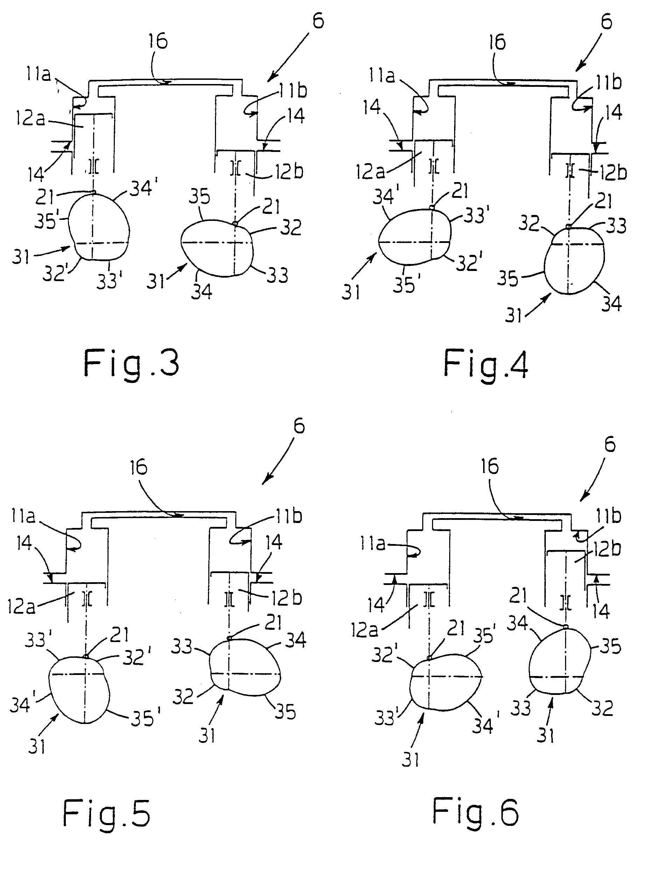

With reference to Figure 2, the motorisation means 13 include an electric motor 27 having a rotating output shaft 28 to which two cams 31 are fitted that are the same as each other but are mounted specularly on the shaft 28 and offset by 90O with respect to each other. The roller 21 of a first piston 12 is in contact with the side surface of a first cam 31, and the roller 21 of the second piston 12 is in contact with the side surface of the second cam 31. The cam 31 (Figure 3) can be subdivided into four sectors, by means of two axes Z' and Z'' at right angles to each other and intersecting at the axis of rotation X of the shaft 28, each of which sectors, on the side surface of the cam 31, is associated with a respective track for contact with the roller 21, which contact arises, for each track, for a quarter turn during the rotation of the cam 31, which rotation is anticlockwise.

Figures 3 to 6 illustrate the stages of operation of the pump 6. Figure 3 illustrates the initial position of the pump 6 in which, in the cylinder 11 connected to the portion 5b (7b), which will be indicated 11a, the related piston, indicated 12a, is at its upper dead point and in which, in the cylinder 11 connected to the portion 5a (7a), which will be indicated 7b, the related piston, indicated 12b, is at its lower dead point. In this initial position, therefore, the mouth 14 of the cylinder 11a is closed by the piston 12a, and the mouth 14 of the cylinder 11b is open. During the rotation of the cam 31 corresponding to the cylinder 11b, the cam 31 is in contact with the roller 21 successively with a first track 32 which causes the piston 12b to stop at the lower dead point, a second track 33 which causes the piston 12b to move from the lower dead point to an intermediate point in which the piston 12b closes the mouth 14, a third track 34 which causes the piston 12b to move from the intermediate point to the upper dead point, and a fourth track 35 which causes the piston 12b to move from the upper dead point to the lower dead point.

During the rotation of the cam 31 corresponding to the cylinder 11a, the cam 31 is in contact with the roller 21 successively with a first track 34' (the same as the track 34) which causes the piston 12a to move from the upper dead point to an intermediate point in which the piston 12a still closes the mouth 14, a second track 33' (the same as the track 33) which causes the piston 12a to move from the intermediate point to the lower dead point, a third track 32' (the same as the track 32) which causes the piston 12a to stop in the lower dead point, and a fourth track 35' (the same as the track 35) which causes the piston 12a to move from the lower dead point to the upper dead point.

It should be emphasised that, in use, the cylinders 11a and 11b and the channel 16 are always full of dialysis liquid. From this initial position (illustrated in Figure 3) it passes, with the shaft 28 rotating by a quarter turn, to a position illustrated in Figure 4 in which, while the piston 12b remains at the lower dead point, the piston 12a moves from the upper dead point to the intermediate point. In this quarter turn, the liquid is sucked from the portion 5a (7a) in a quantity capable of occupying the increase in volume defined in the cylinder 11a; this quantity of liquid enters the cylinder 11b through the mouth 14 and passes from the cylinder 11b through the channel 16 to the cylinder 11a.

From the position illustrated in Figure 4, with the shaft 28 rotating by a further quarter turn, it passes to the position illustrated in Figure 5 in which the piston 12b passes from the lower dead point to the intermediate point in order to close the corresponding mouth 14, and the piston 12a passes from the intermediate point to the lower dead point, opening the corresponding mouth 14; this latter passage occurring at a constant volume. From the position illustrated in Figure 5, with the shaft 28 rotating by a quarter turn, it passes to the position illustrated in Figure 6 in which, while the piston 12a remains at its lower dead point, the piston 12b passes from its intermediate point to the upper dead point. During this movement, there is a reduction in the internal volume of the pump 6 so that the dialysis liquid is compressed, which channels a quantity of liquid, corresponding to the reduction in volume, towards the portion 5b (7b). From the position illustrated in Figure 6, with the shaft 28 rotating by the final quarter turn, it passes to the position illustrated in Figure 3; this latter movement occurring at constant volume.

From what has been described above, it is clear that for each complete rotation (cycle) of the shaft 28 a predetermined quantity of dialysis liquid is first of all sucked from the portion 5a (7a), followed by the emission of the same quantity of liquid in the portion 5b (7b) and, in summary, a pulsating positive flow is generated between the inlet and outlet of the pump 6, with a pulse duration of a quarter cycle. Since the pump 6 is a volumetric pump, the quantity of liquid pumped is very precise. It is now clear that if a central electronic control unit 36 controls the pump 6 installed along the duct 7 to pump a greater quantity of liquid than that pumped by the pump 6 installed in the duct 5, the weight loss of the patient is determined with extreme precision, being the difference between the quantity pumped along the duct 7 and that pumped along the duct 5.

From the above description, the advantages of the invention are clear.

In particular, equipment is produced which, because of the volumetric pump, enables the weight loss of the patient to be controlled with extreme precision since, for this control, it is sufficient to detect the functional characteristics of the electric motors in order to arrive at the flow rates in question. In addition, this control is simple and rapid to execute and can be repeated several times during the dialysis treatment. It should be noted that the equipment is of simple construction and does not require invasive devices for detecting the flow rates that must therefore be sterilised after each treatment. It is therefore clear that further significant advantages of the present invention are that the pump is releasable from the electric motor and that the pump is disposable.

Finally, it is clear that modifications and variations can be introduced into the equipment 1 described and illustrated here without by this departing from the ambit of protection of the present invention.

In particular it is possible to install from two to four pumps 6 in parallel with each other along the duct 5 (7). In this way, with two pumps 6 in parallel, a pulse-like flow with a pulse duration of half a cycle is obtained, with three pumps 6 in parallel, a pulse flow with a pulse duration of three quarters of a cycle is obtained and with four pumps 6 in parallel, a continuous and regular flow is obtained. In addition, as illustrated in Figure 1, the pump 6 can be installed in the duct 9 in order to pump the blood out of the patient or along the duct 10 in order to channel an infusion liquid into the duct 9, downstream of the dialyser 3; these pumps 6 are also controlled by the central control unit 36.

## Claims

1. Equipment for dialysis treatment of the kind including:
a dialyser (3) in which there are two channels delimited by a membrane (4), the first for the extracorporeal blood and the second for a dialysis liquid;
a first duct (5) along which the said dialysis liquid flows towards the said second channel of the said dialyser (3);
first pump means (6) for controlling the flow of the said dialysis liquid along the said first duct (5);
a second duct (7) along which the liquid output from the second channel of the said dialyser (3) flows towards a discharge outlet;
second pump means (6) for controlling the flow of the said dialysis liquid along the said second duct (7);
characterised in that at least one of the said first and second pump means include at least one volumetric pump (6).

2. Equipment according to Claim 1, characterised in that both the said first and second pump means comprise at least one respective volumetric pump (6).

3. Equipment according to Claim 1 and/or Claim 2, characterised in that the said pump (6) has motorisation means (13) that are releasable from the pump (6) itself.

4. Equipment according to Claim 3, characterised in that the said pump (6) is made from plastics material, and is a disposable element, that is, it is intended to be disposed of after each dialysis treatment.

5. Equipment according to Claim 3 and/or Claim 4, characterised in that the said pump (6) includes two cylinders (11) with longitudinal axes parallel to each other, and two pistons (12) which, by means of the said motorisation means (13), slide within the cylinders (11) between an upper dead point and a lower dead point and vice versa; a channel (16) being formed between the said cylinders (11), each of the said cylinders (11) having a mouth (14) at which there is a connection (15) in which is fitted the end of a portion of the said duct (5 or 7) along which the said pump (6) is installed, and the said mouths (14) of the two cylinders (11) are at the same level.

6. Equipment according to Claim 5, characterised in that each of the said pistons (12) have a respective rod (18) which extends out of the related cylinder (11) and supports a rotatable roller (21) at its free end; each of the said pistons (12) being provided with preloaded spring means (24), the action of which consists of pushing the said piston (12) towards the outside of the corresponding said cylinder (11).

7. Equipment according to Claim 6, characterised in that the said motorisation means (13) include a motor (27) having a rotating output shaft (28) to which are fitted two cams (31) that are identical to each other but mounted specularly on the said shaft (28) and offset by 90O with respect to each other; the said roller (21) of a first of the said pistons (12) being in contact on the side surface of the second cam (31), and the said roller (21) of the second of the said pistons (12) being in contact with the side surface of a first cam (31).

8. Equipment according to Claim 7, characterised in that each of the said cams (31) is subdivided into four sectors, by means of two axes (Z' and Z'') at right angles to each other and intersecting at the axis of rotation (X) of the said shaft (28), with each of which sectors, on the side surface of the said cam (31), there is associated a respective track for contact with the said roller (21), which contact occurs for a quarter turn for each track during the rotation of the said cam (31).

9. Equipment according to Claim 8, characterised in that during the rotation of the said first cam (31), it is in successive contact with the respective said roller (21) with a first track (32) which causes the said first piston (21) to stop in the lower dead point in which the corresponding said mouth (14) is open, a second track (33) which causes the said first piston (12) to move from the lower dead point to an intermediate point in which the said first piston (12) closes the corresponding said mouth (14), a third track (34) which causes the said first piston (12) to move from the intermediate point to the upper dead point, and a fourth track (35) which causes the said first piston (12) to move from the upper dead point to the lower dead point.

10. Apparatus according to Claim 9, characterised in that during the rotation of the said second cam (21), it is in successive contact with the corresponding said roller (21) with a first track (34') which causes the said second piston (12) to move from the upper dead point to an intermediate point which the said second piston (12) closes the corresponding said mouth (14), a second track (33') which causes the said second piston (12) to move from the intermediate point to the lower dead point in which the corresponding said mouth (14) is open, a third track (32') which causes the said second piston (12) to stop in the lower dead point, and a fourth track (35') which causes the said second piston (12) to move from the lower dead point to the upper dead point.

11. Equipment according to any preceding claim, characterised in that it includes third pump means similar to the said first and second pump means, provided with at least one volumetric pump (6) installed in a duct (9) for channelling the blood towards the said dialyser (3).

12. Equipment according to any preceding claim, characterised in that it includes fourth pump means similar to the said first and second pump means provided with at least one volumetric pump (6) installed in a duct (10) for channelling the blood towards the patient.

13. Equipment according to any preceding claim, characterised in that it includes a central electronic control unit (13) for controlling the said pump means (6).
